# EUROPEAN PATENT APPLICATION

(11) **EP 4 125 093 A1**
(43) Date of publication of application: **01.02.2023**
(21) Application number: 21187618.0
(22) Date of filing: 26.07.2021
(51) Int. Cl.: G16H 10/60, G16H 20/70, G16H 40/20

(54) **GUIDING A SUBJECT THROUGH A CARE OR TREATMENT SELECTION PROCESS**

(71) Applicant: Koninklijke Philips N.V., 5656 AG Eindhoven (NL)
(72) Inventor: VAN BERKEL, Joep Joseph Benjamin Nathan, 5656 AE Eindhoven (NL); GIL PONCE, Enrique Antonio, 5656 AE Eindhoven (NL); WEIJSEN, Tim Elisabeth Joseph, 5656 AE Eindhoven (NL)
(74) Representative: Philips Intellectual Property & Standards

(57) **Abstract**

A mechanism for aiding a target subject to carry out a sequence of steps, forming a journey, to make a decision or selection about future treatment or care. Historic journey information of similar subjects to the target subject is used to select or decide upon the steps to include in a recommended journey. The operation of a processing system for guiding the tangent subject in carrying out a journey may be made to be responsive to the recommended journey.

## Description

### FIELD OF THE INVENTION

The present invention relates to the field of selecting care or treatment options for a subject.

### BACKGROUND OF THE INVENTION

There is an increasing interest in engaging subjects or patients in their own clinical care decisions. In particular, rather than clinicians making decisions on a subject's behalf a subject based on purely medical or physical considerations, engaging a subject in the clinical decision making process means that other factors important to the subject (such as quality-of-life and desires/fears) are taken into account. This is particularly evident in the rising adoption of holistic care plans.

One area of development is in mechanisms for providing a subject with sufficient information for them to make a clinical decision. For instance, it is known to use systems that process subject information to automatically generate possible treatment options for a patient, and provide the subject with information about these treatment options (e.g. for them to make a decision on how to proceed).

However, this disclosure recognizes that these existing systems can be confusing or overwhelming for a subject, especially if they are inexperienced or unfamiliar with the medical profession and because such decisions are usually made at already stressful times for the subject. It is therefore possible that a subject will not be able to make the most appropriate clinical decision for their needs.

There is therefore a desire to provide mechanisms that increase a likelihood that a subject will make the most appropriate clinical decision for their needs, and that they are able to take account of all necessary information for making the clinical decision.

### SUMMARY OF THE INVENTION

The invention is defined by the claims.

According to examples in accordance with an aspect of the invention, there is provided a computer-implemented method of generating information for guiding a target subject through a care or treatment selection process.

The computer-implemented method comprises: identifying one or more similar subjects to the target subject; obtaining historic journey information comprising, for each similar subject, information about a journey taken by the subject during a care or treatment selection process, wherein a journey is a sequence of steps performed by the subject during a care or treatment selection process; and processing the historic journey information to generate a recommended journey for the target subject.

The present disclosure proposes a new mechanism for guiding or aiding a target subject in making a clinical or treatment decision. In particular, a recommended journey (i.e. procedure or sequence of steps) for making the clinical/treatment decision is generated based on journeys taken by similar subjects (to the target subject). The present invention relies upon a recognition that making a clinical decision is a complex task, and improvements in making a clinical decision can be made by establishing a sequence of steps for the subject to perform in making the clinical decision.

Generating a recommended journey provides the subject with additional information for aiding them in making a most appropriate clinical decision for their needs. In particular, it is recognized that inappropriate journeys will result in the subject not reaching a decision on whether to change or adopt a particular clinical/treatment procedure (i.e. select a treatment/clinical procedure or next step of a treatment or clinical procedure). This may result in the subject being subject to inappropriate medical treatment or potentially not receiving any treatment at all. By guiding the subject through the selection process, there is an increased likelihood that a most appropriate medical treatment for that subject will be provided, i.e. a medical solution or intervention that will address the most pressing (health) needs. Guiding a target subject further through a journey (even if the journey is not successfully completed) improves the target's subject engagement and understanding of the subject, thereby increasing a likelihood that the target subject will seek out medical/clinical help when required.

The step of processing the historic journey information comprises: defining a plurality of stages for the journey, wherein each stage can be performed using one or more alternative steps; selecting, for each stage of the journey, one of the one or more alternative steps to be performed to carry out that stage of the journey by using the historic journey information to select one of the alternative steps; and defining the recommended journey as a sequence of the selected steps.

A journey may be conceptually divided into a number of stages. Each stage may be achieved or performed using a number of alternatives. The step of generating the recommended journey may comprise selecting one of these alternatives for each stage, to thereby form the sequence of steps that forms the journey.

In some examples, the recommended journey may be formed from a sequence of default steps ("default sequence"), each of which has one or more alternative steps. The historic journey information may be used to switch a step (of the default sequence) to an alternative step based on the historic journey information (e.g. if similar subjects are more successful using the alternative step).

In some examples, the historic information comprises, for each similar subject, an identification of each step taken by the similar subject during the care or treatment selection process; and the step of processing the historic journey information comprises constructing a recommended journey for the target subject using the steps taken by the identified similar subject.

Thus, the steps recommended to the target subject can be based on those taken by similar subjects during their decision making process. This increases a likelihood that the recommended journey will be one that was successful amongst their peers.

In some examples, the historic information further comprises, for each similar subject, an identification of any assistance used to aid the subject in the completion of any of the steps taken by the subject during the care or treatment selection process; and the step of processing the historic journey information comprises recommending assistance for steps of the recommended journey based on the identified assistance used to aid any of the similar subjects.

The assistance used to aid the subject in the completion of any of the steps may comprise: assistance or guidance from a clinician; assistance or guidance from a caregiver; and/or provision of additional information for guiding the subject through a step of the journey.

In some embodiments, the historic journey information further comprises, for each similar subject, a measure of success of each step taken by the subject; the step of processing the historic journey information comprises processing the measure of success of each step taken by the similar subject to construct the recommended journey for the target subject.

The step of processing the historic journey information may comprise: identifying, in the historic journey information, one or more barrier steps by processing the measure of success of each step, wherein barrier steps are steps that are more likely to be challenging for the target subject to complete; and generating a recommended journey by processing the identified barrier steps.

A barrier step may, for instance, be one that similar subjects have taken a long time to perform (e.g. an average time period to perform the step is more than a predetermined average time period), required additional assistance to perform (e.g. more than a predetermined number/percentage (e.g. 50%) of similar subjects have required assistance) and/or have been unable to perform (e.g. more than a predetermined number/percentage (e.g. 30%, 40%, 50% or 60%) of similar subjects have not been able to complete).

The step of generating the recommended journey may comprise generating a recommended journey that avoids one or more of any identified barrier step and/or recommends additional assistance for one or more of any identified barrier steps.

Optionally, the historic information further comprises, for each similar subject, an overall success indicator of whether the journey taken by the similar subject was successful; and the step of processing the historic journey information comprises processing the overall success indicator to construct the recommended journey for the target subject.

A successful journey is one in which the subject makes a decision to change a care or treatment approach or to select a care or treatment approach to be applied.

Optionally, the step of identifying one or more similar subjects to the target subject comprises: obtaining characteristic information of the target subject, comprising information on one or more characteristics of the target subject; and processing the characteristic information to identify one or more similar subjects that share similar characteristics to the target subject.

In at least one example, the one or more characteristics of the target subject comprise one or more of: a level of engagement of the target subject, the needs of the target subject, an aimed value of care for the target subject, a susceptibility to conversion of the target subject, a cost of care available to the target subject, and/or informal caregiver availability and involvement.

The method may further comprise a step of providing, at a user interface, a visual representation of the recommended journey.

There is also proposed a computer program product comprising computer program code means which, when executed on a computing device having a processing system, cause the processing system to perform all of the steps of any herein described method.

There is also proposed a processing system for generating information for guiding a target subject through a care or treatment selection process. The processing system is configured to: identify one or more similar subjects to the target subject; obtain historic journey information comprising, for each similar subject, information about a journey taken by the subject during a care or treatment selection process, wherein a journey is a sequence of steps performed by the subject during a care or treatment selection process; and process the historic journey information to generate a recommended journey for the target subject.

The processing system may be configured to perform any herein described method, just as any herein described method may be configured to carry out the functions performed by any herein described processing system and/or system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system in which embodiments may be employed;
Figure 2 illustrates a method according to an embodiment;
Figure 3 illustrates a process for use in a method;
Figure 4 illustrates another process for use in a method;
Figure 5 illustrates a method according to an embodiment;
Figure 6 illustrates a processing system; and
Figure 7 illustrates a system comprising a processing system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for aiding a target subject to carry out a sequence of steps, forming a journey, to make a decision or selection about future treatment or care. Historic journey information of similar subjects to the target subject is used to select or decide upon the steps to include in a recommended journey. The operation of a processing system for guiding the tangent subject in carrying out a journey may be made to be responsive to the recommended journey.

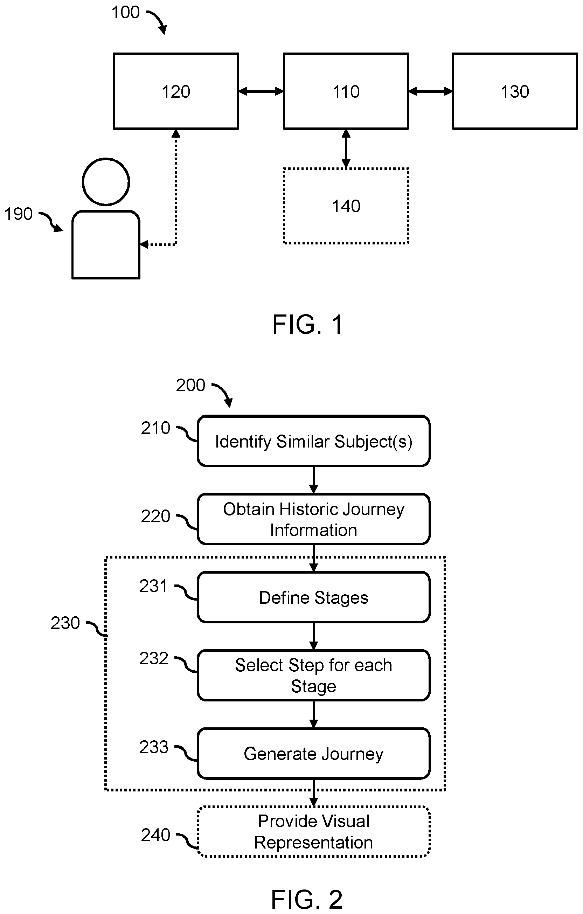

Figure 1 illustrates a system 100 in which embodiments may be implemented for improved contextual understanding.

The system 100 comprises a processing system 110, an input user interface 120, with which a target subject 190 may interact in order to provide input to the processing system and an output user interface 130, which is controlled by the processing system to provide a user-perceptible output (e.g. a visual, audio and/or haptic output). The input and output user interface may be together referred to as a "user interface".

The input user interface and the output user interface may be integrated together as a single combined user interface, e.g. a touch-sensitive screen or the like. Alternatively, the input user interface and the output user interface may be provided separately (e.g. in the form of a keyboard and/or mouse and a display screen). Other suitable combinations will be readily apparent to the skilled person, e.g. a touchscreen display and an alternative input user interface.

The processing system 110 and user interfaces 120, 130 may be integrated into a single device, such as a mobile/cellular (smart) phone or tablet. Other suitable examples of processing systems will be apparent to the skilled person.

The processing system 110 is configured to interact with the target subject via the user interfaces 120, 130 in order to guide the target subject 190 through a care/treatment selection process. This may comprise, for example, allowing the user to select information about their condition, treatment options, likely outcomes and so on, which is then presented to the user at the output user interface. In some examples, the processing system 110 only performs this operation when running a particular program or application.

The processing system 110 may be able to communicate with a memory system 140 storing information about the subject and/or their selection choices, in order to retrieve and provide desired information to the subject. The memory system 140 may be an external memory, e.g. a cloud-storage system or the like.

When making the care/treatment selection process, the target subject 190 progresses through a sequence of steps/actions (or a "journey") in order to make a decision. During a journey, the target subject will learn and/or reflect upon information about their condition, their needs/desires, treatment options, potential outcomes and/or other information that is used to make a decision. Each step or action may represent a user reviewing or understanding a different piece of information (e.g. provided by the processing system via the output user interface or via introspection).

Different steps of a journey may represent different actions for monitoring, reviewing, identifying or understanding different pieces of information that a user should process in order to make a decision. For instance, one step may be to identify diagnosis information, a second step may be to reflect upon diagnosis information and a third step may be to understand diagnosis information. Yet another step may be to identify one or more possible treatment options, with a further step comprise reflecting upon the different treatment options. Other suitable examples of steps or actions performed in the process of making a clinical or treatment decision will be apparent to the skilled person.

The processing system 110 aids the target subject in the performance of the journey, e.g. by providing information in response to a user request to facilitate understanding of information necessary for completing a step of the journey.

In this way, the target subject 190 may interact with the processing system 110 during performance of a target journey, and thereby view and obtain data for aiding them in the execution of different steps of the target journey. For instance, a target subject 190 may request (via the user interface 120) information on their current condition, which may be provided (via the output interface 130) by the processing system.

Successful completion of a journey by a subject means that the subject has (when the journey is complete) been able to make a care/treatment plan decision. Examples of care/treatment plan decisions include a decision to visit a clinical environment (e.g. visit the GP), a decision to pursue a particular course of treatment or care, a decision on a medication, surgical intervention or other treatment option to take and so on. A care-treatment plan decision may be a decision to take a particular step in a larger care/treatment plan (e.g. a decision to take a diagnostic test or the like). Similarly, an incomplete journey is one in which the subject does not make a care/treatment plan decision (or change to care/treatment plan).

The present disclosure proposes an approach for generating a recommended journey for the target subject, i.e. in order to identify and recommend steps that the subject should take in order to successfully complete a journey and make a decision.

The recommended journey may be used by the processing system 110 to suggest next steps for the subject (e.g. based on the progress of the subject through the recommended journey). In some examples, a visual representation of the recommended journey is presented to the subject (e.g. at the output interface 130) so that the subject is able to understand and reflect upon their progress through the recommended journey and upcoming steps. In some examples, a visual representation of the next step of the recommended journey may be presented to the user.

The present disclosure is based on the realization that completion of a journey is important in ensuring that the target subject selects a care or treatment (plan). This reduces the likelihood that no, or an inappropriate, care or treatment plan will be provided to the target subject.

It is also recognized that partial completion of a journey can help ensure that a target subject gets necessary care/treatment (e.g. if a step involves reaching out to a clinician or family member). Thus, there is a strong motivation to aid a target subject in progressing through the steps of a journey.

It is further recognized that every target user will have different needs, different levels of engagement and involvement, health literacy, awareness and different levels of support such as family helper contributions. Thus, a journey that works for one subject will not necessarily be as effective for the target subject. For instance, one subject may have substantial familial support, and completion of their journey may be reliant upon familial support. Whereas another subject may have less familial support, so that likely completion of their journey would be significantly reduced if steps required significant familial support.

Embodiments according to the present disclosure recognize that guidance can be provided to aid a target subject's journey (to care/treatment selection) based on journeys taken by other (similar) subjects to the target subject. In this way, an appropriate journey can be identified and actions taken to guide the user on carrying out the steps for completing this journey.

Figure 2 illustrates a (computer-implemented) method 200 according to an embodiment. The method may be performed by a processing system, such as the processing system 110 of Figure 1.

The method 200 comprises a step 210 of identifying one or more similar subjects to the target subject. Mechanisms for identifying similar subjects are well established in the art, such as that proposed in international patent application no. PCT/EP2019/078701.

A "similar subject" may be a subject who shares one or more characteristics with the target subject, e.g. one or more demographic characteristics, diagnosis information, health care payer claims based data, heathcare utilisation, social determinants of health data. Other approaches for identifying similar subjects will be apparent to the skilled person.

In some examples, a similar subject may be a subject belonging to a same cluster of subjects as the target subject. In particular, a population/plurality of subjects (including the target subject) may undergo a clustering algorithm (e.g. a k-means clustering algorithm, a density-based clustering algorithm, a distribution based clustering algorithm and so on). The clustering algorithm processes characteristics of the subjects (e.g. demographic information such as age, gender and so on, and/or medical information such as diagnosis, treatments applied, medical history and so on) in order to identify clusters of subjects, as well established in the art. The identified one or more similar subjects may be the subjects belonging to a same cluster as the target subject.

In one example, step 220 comprises obtaining characteristic information of the target subject, comprising information on one or more characteristics of the target subject; and processing the characteristic information to identify one or more similar subjects that share similar characteristics to the target subject.

The one or more characteristics of the target subject may comprise one or more of: a level of engagement of the target subject, the needs of the target subject, an aimed value of care for the target subject, a susceptibility to conversion of the target subject, a cost of care available to the target subject, and/or informal caregiver availability and involvement. Of course, other characteristics of the target subject, e.g. socio-econimic data, medical data, underlying conditions or illnesses information, adherence-to-therapy data and so on could be used as characteristics.

By way of example, a level of engagement may be obtained by monitoring the target subject's interaction(s) with information provided at a user interface. For instance, a level of engagement may be determined by processing a length of time that a user spends reading information and/or an amount of information accessed by the subject (both of which indicate engagement of the subject with information provided to them.

In some examples, interaction patterns of a target subject with information provided at the user interface may be processed in order to assess a level of engagement of the target subject.

Some characteristics of the subject may be derived from responses to a questionnaire provided to the subject, e.g. so that the subject may indicate their needs and/or aimed value of cares.

Information on informal caregiver availability and involvement may be obtained, for instance, by receiving information input by the informal caregiver and/or the target subject and/or by automatically processing calendars or availability information of the informal caregivers.

Other forms of characteristics will be readily apparent to the skilled person, and may include medical history information, diagnosis, treatment information, physiological data, demographic information (e.g. age, gender, weight and so on).

The method 200 then moves to a step 220 of obtaining historic journey information comprising, for each similar subject, information about a journey taken by the subject during a care or treatment selection process. As previously explained, a journey is a sequence of steps performed by the subject during a care or treatment selection process.

Step 220 may comprise obtaining the historic journey information from a memory or database. Approaches for obtaining data from a memory are well-established, and may comprise providing a request for the historic journey information to the memory or databased and receiving the historic journey information from the database.

The method 200 then performs a step 230 of processing the historic journey information to generate a recommended journey for the target subject. Thus, the historic journey information may be used to define the recommended journey for the target subject.

In particular examples, step 230 may comprise generating recommended journey data that comprises one or more step indicators (e.g. formed as a sequence of step indicators), each step indicator being a data element that identifies a step to be performed by a subject to carry out the recommended journey for the subject. Each step indicator may point to a preceding and/or following step indicator in a sequence. The step indicators may thereby provide, for a processing system, information about the steps of a recommended journey (e.g. so that the processing system can suggest the recommended journey and/or next steps for a target subject undergoing a journey).

In some examples, the recommended journey data may comprise, for one or more step indicators, supplementary data including additional recommendations for aiding in the performance of the step associated with the step indicator. The supplementary data may comprise, for instance, an indicator of a recommended assistance to provide to the target subject whilst the target subject carries out that recommended step. This supplementary data provides information for a processing arrangement to guide the performance of a step.

The method 200 may comprise a step 240 of providing, at a user interface, a visual representation of the recommended journey. Thus, step 240 may comprise controlling a user interface to provide a visual representation of the recommended journey.

One example for step 230 is hereafter described, with other examples provided later in the disclosure.

Step 230 may comprise, for instance, a sub-step 231 defining a plurality of stages for the journey, wherein each stage can be performed using one or more alternative steps. In this way, a journey can be defined as a sequence of stages, in which completion of stage can be achieved using different or alternative steps. For instance, an example stage of learning about a particular treatment may be performed by carrying out a step of: talking to a clinician, reading an article, discussing with friends/family, listening to a lecture and so on.

The stages for a journey may be predefined, e.g. according to some predetermined sequence of stages for aiding a user in performing a journey. The precise stages performed to complete a journey are not important to the present invention, and the skilled person would readily understand how different stages may be carried out in order to complete a journey according to some.

Step 230 may then move to a sub-step 232 of selecting, for each stage of the journey, one of the one or more alternative steps to be performed to carry out that stage of the journey by using the historic journey information to select one of the alternative steps.

In particular, the historic journey information may comprise an indication of the step performed by each similar subject to carry out each stage of the journey (where the step is one of the alternative steps). The historic journey information may also indicate whether the journey was successfully completed (e.g. whether the similar subject was able to make a care/treatment plan decision), which information can be used to discriminate or select between different steps. In some instances, the alternative steps may be defined by the historic journey information, i.e. by the steps taken by other subjects during their journeys.

As one example, step 232 may comprise selecting the most common step used for that stage by the similar subjects (as defined in the historic journey information) as the step to take for that stage. As another example, step 232 may comprise selecting the most common step used for that stage by only those similar subjects whose journey was successful.

Other approaches may use additional information about the target subject and/or the similar subjects in order to select a step for the subject to carry out a particular stage. For instance, a most similar subject to the target subject (who successfully completed the journey) may be identified, and the step used by the most similar subject selected as the step for that stage of the journey.

Step 230 may then perform a sub-step 233 of defining the recommended journey as a sequence of the selected steps. In this way, a recommended journey is built up or constructed based on historic journey information, and in particular, using the historic journey information to select between different alternatives for a same stage of the journey.

Sub-step 233 may comprise, for instance, generating a sequence of step indicators, each step indicator indicating a recommended step to be performed to complete a stage of the journey.

Step 230 may therefore comprise constructing a recommended journey for the target subject using the steps taken by the identified similar subject. In particular, the historic information may comprise, for each similar subject, an identification of each step taken by the similar subject during the care or treatment selection process.

In some examples, step 230 may be modified to comprise avoiding performance of one or more stages responsive to the historic journey information.

Other approaches for constructing a recommended journey based on such historic information (i.e. rather than selecting a step for each stage) could be performed. For instance, step 230 may comprise selecting a journey taken by a most similar subject (who successfully completed the journey) as the recommended journey, which is thereby formed from the sequence of steps of the journey of the most similar subject.

In some examples, the historic information further comprises, for each similar subject, an identification of any assistance used to aid the subject in the completion of any of the steps taken by the subject during the care or treatment selection process.

In this example, step 230 may further comprise recommending assistance for steps of the recommended journey based on the identified assistance used to aid any of the similar subjects.

Assistance may include, for instance, using a/the processing system to provide additional information or explanations to the target subject during performance of a particular step and/or connecting a clinician or caregiver to the subject during performance of a particular step (e.g. to provide emotional aid and/or additional (e.g. expert) information). More generally, the assistance may comprise: assistance or guidance from a clinician; assistance or guidance from a caregiver; and/or provision of additional information for guiding the subject through a step of the journey.

Thus, step 230 may effectively further comprise identifying whether additional assistance would be helpful in completing one or more steps of the recommended journey (based on assistance provided to one or more similar subjects). Any additional assistance provided to the similar subject(s) is used to determine whether to provide the additional assistance to the target subject.

In some examples, the historic journey information further comprises, for each similar subject, a measure of success of each step taken by the subject.

A measure of success may, for instance, be a numeric value responsive to a length of time taken to perform the step, a number of times the step is repeated, whether or not the step takes a minimum predetermined period of time (e.g. indicating that the subject has successfully completed the step) and so on. Thus, a measure of success may be a measure responsive to any parameter that indicates whether the subject has successfully completed the step.

By way of further example, a measure of success for a particular step may be derived, for instance, by processing information on whether the step was successfully completed by the subject, a time spent on completing the step, a level of engagement of the subject with the step and the an indicator of success of the journey (including the step).

As a simple example, a measure of success for a step performed by a subject may be "1" or "0", dependent upon whether or not the step was successfully completed by the subject.

As another example, a measure of success for a step performed by a subject may be "1" or "0", dependent upon whether or not the journey including the step was successfully completed by the subject.

As yet another example, a measure of success for a step performed by a subject may be on a scale of 0 to 1, where 0 indicates failure to complete a step or that completion of the step took more than a first predetermined period of time and 1 indicates that the step was completed in less than a second (different) predetermined period of time, in which values between 0 to 1 are set on a sliding scale between the first and second predetermined periods of time.

Various other examples will be readily apparent to the skilled person.

In some examples, step 230 may comprise processing the measure of success of each step taken by the similar subject to construct the recommended journey for the target subject. This may comprise, for instance, identifying steps taken by a set of one or more similar subjects that had a highest (average) level of success as steps to include in the recommended journey.

The measure of success for each step performed by the similar subject(s) can thereby be used as an indicator as to whether or not that step should be included in the recommended journey and/or whether or not additional assistance should be provided when the target subject is performing the step.

For instance, where step 230 is performed by executing sub-steps 231, 232 and 233, then step 232 may comprise selecting the step having the highest (average) level of success for the similar subject(s).

As another example, where step 230 comprises recommending assistance for steps of the recommended journey based on the identified assistance used to aid any of the similar subjects, then step 230 may comprise recommending assistance to be provided only if the selected step has a success level below some predetermined threshold. This approach reduces the processing power and/or clinician input required for a step to be completed, e.g. by avoiding providing additional assistance if it is unlikely that the subject will require the information to complete the journey.

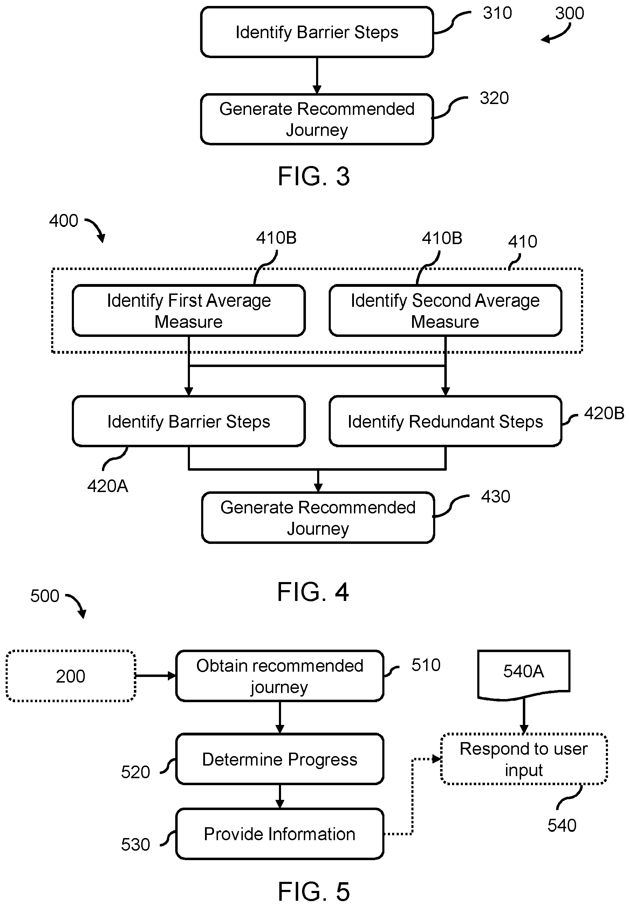

Figure 3 illustrates a process 300, which can be used to perform step 230 of method 200. For process 300, the historic journey information further comprises, for each similar subject, a measure of success of each step taken by the subject.

The process 300 comprises a step 310 of identifying, in the historic journey information, one or more barrier steps by processing the measure of success of each step. Barrier steps are steps that are more likely to be challenging for the target subject to complete, i.e. steps that impact the execution and likely completion of a journey by the subject.

A measure of success for a particular step may be derived, for instance, by processing information on whether the step was successfully completed by the subject, a time spend on completing the step, a level of engagement of the subject with the step and the an indicator of success of the journey (including the step).

Identification of a barrier event may comprise, for instance, identifying steps associated with a measure of success below some predetermined level, e.g. steps that take more than a predetermined length of time to complete or are not completed by more than a predetermined percentage of similar subjects.

In one example, identification of a barrier step may comprise identifying incomplete journeys (of similar patients) and identifying the final step of the incomplete journey as a barrier step.

The process 300 may then perform a step 320 of generating a recommended journey by processing the identified barrier steps. In particular, step 320 may comprise generating a recommended journey that avoids one or more of any identified barrier step and/or recommends additional assistance for one or more of any identified barrier steps.

By way of example, step 320 may comprise selecting an alternative step to the barrier step that achieves the same outcome as the barrier step. This increases a likelihood that the target subject (following the recommended journey) will be able to successfully complete the journey.

As another example, step 320 may comprise recommending additional assistance if a barrier step is included in the recommended journey, e.g. recommending that additional information is provided to the subject and/or a clinician/caregiver aids in the performance of a barrier step.

Thus, generation of a recommended journey may comprise diverting around potential barrier steps and/or recommending that additional assistance is provided along with the recommended journey to overcome barrier steps.

In one example, step 320 is performed by carrying out sub-steps 231, 232, 233 as previously described, in which sub-step 232 comprises avoiding selection of a barrier step in order to perform a stage for the recommended journey.

Step 320 may comprise, for instance, generating supplementary information to accompany one or more step indicators of a sequence of step indicators. The sequence of step indicators may be generated, for example, by performing the sub-steps 231, 232, 233 as previously described. The supplementary information may be associated with step indicators that indicate barrier steps to be performed, and may indicate a need to provide additional assistance for aiding the target subject in carrying out the indicated step.

Figure 4 illustrates a process 400 that provides another example of step 230 of method 200. For process 400, the historic journey information further comprises, for each similar subject, a measure of success of each step taken by the subject.

The process 400 comprises a step 410 of processing the historic journey information to identify, for each step, at least one average measure of success for that step.

In particular, step 410 comprise a sub-step 410A of identifying, for each step, a first average measure, being an average measure of success for that step for only subjects that successfully completed the journey. Similarly, step 410 may comprise a sub-step 410B of identifying, for each step, a second average measure, being an average measure of success for that step for subjects that did not successfully complete the journey.

The process 400 may further comprise a step 420A of identifying any steps for which the first average measure exceeds a first predetermined average threshold and for which the second average measure falls below a second predetermined average threshold. In this way, step 420A effectively identifies any "barrier steps" (i.e. steps that are likely to contribute to completion of the journey but which are difficult to perform).

The process 400 may further comprise a step 420B of identifying any steps for which: the first average measure falls below a third predetermined average threshold. In this way, step 420A effectively identifies any "superfluous steps" or "redundant steps" (i.e. steps that are unlikely to contribute to successful completion of the journey).

The process 400 then performs a step 430 of constructing a recommended journey based on the historic information. As previously explained, this may comprise generating recommended journey information comprise identifiers for steps for the target subject to perform.

Step 430 may comprise excluding, from the recommended journey, any steps that are identified as "superfluous steps". In this way, one or more steps performed by similar subjects can be excluded from the recommended journey.

Step 430 may comprise, if any barrier steps are identified, including supplementary data for recommending additional assistance is provided to the target subject when carrying out the barrier step.

In this way, a journey can be constructed that includes steps that were successful in progressing similar subjects to completion, whilst avoiding inclusion of steps that do not help the subject in progressing to completion.

The generated recommended journey may be used, e.g. by the processing system, in order to aid a target subject in carrying out the recommended journey. In particular, guidance and/or information may be controlled and provided to the target subject to aid them in completion of different steps of the recommended journey.

In particular, the operation of the processing system (which is used by the target subject to perform a journey) is responsive to recommended journey.

Figure 5 illustrates a method according to an embodiment. The method 500 is performed by a processing system.

The method 500 comprises a step 510 of obtaining a recommended journey of a target subject. The recommended journey may be a journey generated by performing the method 200 previously described. Step 510 may comprise performing method 200 or retrieving a previously generated recommended journey (e.g. from storage).

Step 510 may comprise obtaining recommended journey data, which comprises step indicators and optionally supplementary data for one or more of the step indicators.

The method 500 then performs a step 520 of determining a current progress of the target subject through the recommended journey. In this way, step 520 effectively identifies a next step in the recommended journey for the target subject.

A current progress effectively identifies a most recently completed step of the recommended journey that has been carried out by the subject. Of course, if the target subject has not yet started a journey this step may comprise identifying that the target subject has not yet started the journey.

Step 520 may be performed by obtaining historic interaction data, e.g. from a memory, that identifies previous interactions of the user with the processing system during performance of a journey. The historic interaction data can be processed to identify a current progress through the recommended journey.

The method 500 further comprises a step 530 of providing information to a subject responsive to the next step in the recommended journey.

Step 530 may comprise, for example, providing a visual representation of the next step in the recommended journey (e.g. to prompt the subject to perform the next step of the recommended journey) and/or providing additional assistance to guide the target subject in performing the next step (e.g. providing information for aiding the subject in performing the next step and/or highlighting information to be used in a next step).

Thus, the recommended journey may be used, for instance, to automatically provide information that would aid a user in performing a next step of the recommended journey.

In some examples, step 530 comprises identifying and performing, based on any supplementary data associated with the next step of the recommended journey, one or more actions to aid a user in carrying out the next step of the recommended journey.

For instance, if the supplementary data for a next step indicates that clinical guidance is recommended, then step 530 may comprise providing a visual representation indicating that clinical guidance is recommended to aid the target subject in performing the step and/or connecting the target subject to a clinician (e.g. via a mobile telephone communication channel and/or wireless communication channel) to allow the clinician to guide the target subject in performing the next step.

Method 500 may be repeated a number of times, e.g. each time a subject completes a step. In each iteration, step 510 may be repeated, and in particular a new recommended journey may be generated (e.g. by repeating method 200). This allows new characteristics of the target subject (e.g. characteristics that change responsive to how the target subject perform a previous step) to be used to identify new similar subjects, and therefore potential new routes for performing the recommended journey.

Method 500 may further comprise a process 540 of responding to a user input 540A in order to provide information (e.g. in the form of a visual representation) to aid/guide the user in performing a (current) step of a journey and/or to evidence performance or completion of a particular step.

For instance, a current step may be to "discover more about your medical condition", and the user may select or investigate different effects of their medical condition (e.g. by selecting summaries or identifiers of the effects), in response to which process 540 provides information about the selected effect.

As another example, a current step may be to "reflect upon your needs and desires", and the user may select or input one or more needs or desires to the processing system (e.g. via an input user interface), to thereby indicate completion of the step.

The process 540 may be modified responsive to the recommended journey (i.e. step 530 may comprise modifying the process 540). For instance, the amount of information provided responsive to a user input may be responsive to a recommended step. As another example, the target subject may be provided with a visual indicator of whether or not the displayed information is considered helpful for performing a next recommended step of the recommended journey (e.g. to prompt or guide the target subject into obtaining information useful for performing a next recommended step).

Information on the steps taken by the target subject and optionally a success measure of each step may be stored. This information may be stored for use with future target subjects (i.e. be added to the historic journey information).

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

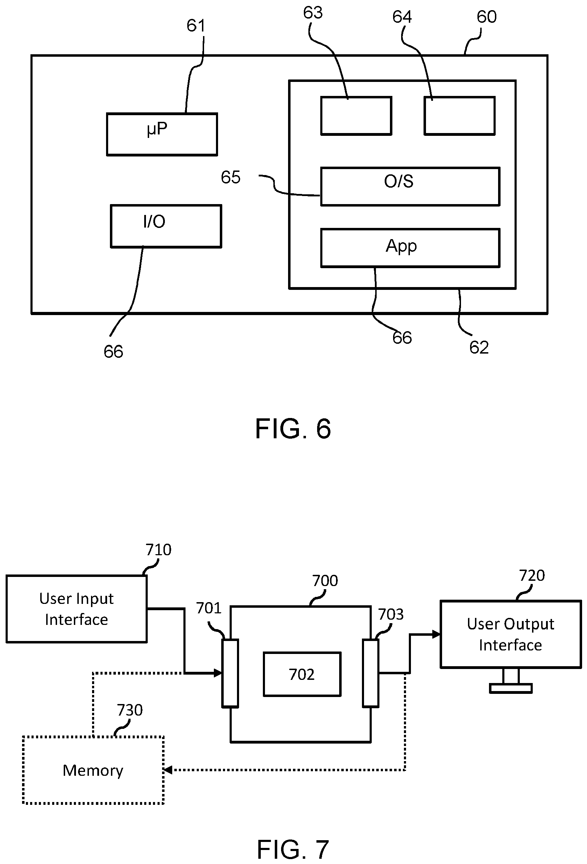

By way of further example, Figure 6 illustrates an example of a processing system 60 within which one or more parts of an embodiment may be employed. Various operations discussed above may utilize the capabilities of the processing system 60. For example, one or more parts of a system estimating a relevance of sections of unstructured textual data of a medical record may be incorporated in any element, module, application, and/or component discussed herein. In this regard, it is to be understood that system functional blocks can run on a single computer or may be distributed over several computers and locations (e.g. connected via internet).

The processing system 60 includes, but is not limited to, PCs, workstations, laptops, PDAs, palm devices, servers, storages, and the like. Generally, in terms of hardware architecture, the processing system 60 may include one or more processors 61, memory 62, and one or more I/O devices 67 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 61 is a hardware device for executing software that can be stored in the memory 62. The processor 61 can be virtually any custom made or commercially available processor, a central processing unit (CPU), a digital signal processor (DSP), or an auxiliary processor among several processors associated with the processing system 60, and the processor 61 may be a semiconductor based microprocessor (in the form of a microchip) or a microprocessor.

The memory 62 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 62 may incorporate electronic, magnetic, optical, and/or other types of storage media. Note that the memory 62 can have a distributed architecture, where various components are situated remote from one another, but can be accessed by the processor 61.

The software in the memory 62 may include one or more separate programs, each of which comprises an ordered listing of executable instructions for implementing logical functions. The software in the memory 62 includes a suitable operating system (O/S) 65, compiler 64, source code 63, and one or more applications 66 in accordance with exemplary embodiments. As illustrated, the application 66 comprises numerous functional components for implementing the features and operations of the exemplary embodiments. The application 66 of the processing system 60 may represent various applications, computational units, logic, functional units, processes, operations, virtual entities, and/or modules in accordance with exemplary embodiments, but the application 66 is not meant to be a limitation.

The operating system 65 controls the execution of other computer programs, and provides scheduling, input-output control, file and data management, memory management, and communication control and related services. It is contemplated by the inventors that the application 66 for implementing exemplary embodiments may be applicable on all commercially available operating systems.

Application 66 may be a source program, executable program (object code), script, or any other entity comprising a set of instructions to be performed. When a source program, then the program is usually translated via a compiler (such as the compiler 64), assembler, interpreter, or the like, which may or may not be included within the memory 62, so as to operate properly in connection with the O/S 65. Furthermore, the application 66 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 67 may comprise at least an input interface. The input interface is configured to obtain or receive data to the processing system and may, for instance, be configured to obtain user input (received at a user input interface) and/or information about the target subject and/or information responsive to a user input.

The I/O devices 67 may also comprise an output interface, which is configured to provide data for controlling an external device or passing to an external device. By way of example, the output interface may provide the recommended journey (data) and/or provide display data for controlling a display to provide a visual representation of the recommended journey and/or a next step in a recommended journey, optionally as well as information responsive to a user input or enquiry.

If the processing system 60 is a PC, workstation, intelligent device or the like, the software in the memory 62 may further include a basic input output system (BIOS) (omitted for simplicity). The BIOS is a set of essential software routines that initialize and test hardware at startup, start the O/S 65, and support the transfer of data among the hardware devices. The BIOS is stored in some type of read-only-memory, such as ROM, PROM, EPROM, EEPROM or the like, so that the BIOS can be executed when the processing system 60 is activated.

When the processing system 60 is in operation, the processor 61 is configured to execute software stored within the memory 62, to communicate data to and from the memory 62, and to generally control operations of the processing system 60 pursuant to the software. The application 66 and the O/S 65 are read, in whole or in part, by the processor 61, perhaps buffered within the processor 61, and then executed.

When the application 66 is implemented in software it should be noted that the application 66 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 66 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Figure 7 illustrates a system 70 according to an embodiment.

The system 70 comprises a processing system 700, which may be embodied as the processing system 60 described with reference to Figure 6. Other suitable examples of a processing system will be apparent to the skilled person.

The system 70 may form, for instance, a (smart)phone, tablet, laptop, computer and so on.

The system 70 may also comprise an input user interface 710 and/or an output user interface 720.

The processing system 700 may comprise an input interface 701, configured to obtain input from the input user interface and/or communicate with a (optional) memory or storage system 730 of the system 70.

The processing system 700 may comprise a processor 702 configured to identify one or more similar subjects to the target subject; obtain historic journey information comprising, for each similar subject, information about a journey taken by the subject during a care or treatment selection process, wherein a journey is a sequence of steps performed by the subject during a care or treatment selection process; and process the historic journey information to generate a recommended journey for the target subject.

The recommended journey can then be used to guide a target subject through performing the journey. In particular, the processing system may be configured to provide a visual representation of the recommended journey and/or a next step in the recommended journey (e.g. by controlling the output user interface via an output interface 703).

The processing system 700 may also be configured to respond to input signals received from the input user interface to provide (at the output user interface) a visual representation of information responsive to a target subject's enquiry. This effectively helps guide a user through a journey.

The recommended journey (data) may include supplementary information indicating for which steps additional assistance should be provided (and optionally, what the additional assistance should contain). The processing system 700 may, when a user it performing a step for which additional assistance is indicated, provide such additional assistance, e.g. providing additional information for the user's understanding and/or provide a recommendation to contact a clinician and/or (informal) caregiver.

The processing system may repeatedly generate a new recommended journey as the target subject progresses through the journey, e.g. to take into account success and/or failure of the target subject to perform certain steps of a previously recommended journey.

The processing system may store a generated recommended journey 730 and/or information responsive to actions of the user in the memory 730. The stored actions may form part of historic journey information.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (200) of generating information for guiding a target subject (190) through a care or treatment selection process, the computer-implemented method comprising:
identifying (210) one or more similar subjects to the target subject;
obtaining (220) historic journey information comprising, for each similar subject, information about a journey taken by the subject during a care or treatment selection process, wherein a journey is a sequence of steps performed by the subject during a care or treatment selection process; and
processing (230, 300, 400) the historic journey information to generate a recommended journey for the target subject.

2. The computer-implemented method (200) of claim 1, wherein the step (230) of processing the historic journey information comprises:
defining (231) a plurality of stages for the journey, wherein each stage can be performed using one or more alternative steps;
selecting (232), for each stage of the journey, one of the one or more alternative steps to be performed to carry out that stage of the journey by using the historic journey information to select one of the alternative steps; and
defining (233) the recommended journey as a sequence of the selected steps.

3. The computer-implemented method (200) of claim 1 or 2, wherein:
the historic information comprises, for each similar subject, an identification of each step taken by the similar subject during the care or treatment selection process; and
the step of processing the historic journey information comprises constructing a recommended journey for the target subject using the steps taken by the identified similar subject.

4. The computer-implemented method of claim 3, wherein:
the historic information further comprises, for each similar subject, an identification of any assistance used to aid the subject in the completion of any of the steps taken by the subject during the care or treatment selection process; and
the step of processing the historic journey information comprises recommending assistance for steps of the recommended journey based on the identified assistance used to aid any of the similar subjects.

5. The computer-implemented method of claim 4, wherein the assistance used to aid the subject in the completion of any of the steps comprises: assistance or guidance from a clinician; assistance or guidance from a caregiver; and/or provision of additional information for guiding the subject through a step of the journey.

6. The computer-implemented method of any of claims 3 to 5, wherein:
the historic journey information further comprises, for each similar subject, a measure of success of each step taken by the subject;
the step of processing the historic journey information comprises processing the measure of success of each step taken by the similar subject to construct the recommended journey for the target subject.

7. The computer-implemented method of claim 6, wherein the step (300, 400) of processing the historic journey information comprises:
identifying (310, 420A), in the historic journey information, one or more barrier steps by processing the measure of success of each step, wherein barrier steps are steps that are more likely to be challenging for the target subject to complete; and
generating (320, 430) a recommended journey by processing the identified barrier steps.

8. The computer-implemented method of claim 7, wherein the step (320, 430) of generating the recommended journey comprises generating a recommended journey that avoids one or more of any identified barrier step and/or recommends additional assistance for one or more of any identified barrier steps.

9. The computer-implemented method of any of claims 1 to 8, wherein:
the historic information further comprises, for each similar subject, an overall success indicator of whether the journey taken by the similar subject was successful; and
the step of processing the historic journey information comprises processing the overall success indicator to construct the recommended journey for the target subject.

10. The computer-implemented method of any of claims 1 to 9, wherein the step of identifying one or more similar subjects to the target subject comprises:
obtaining characteristic information of the target subject, comprising information on one or more characteristics of the target subject; and
processing the characteristic information to identify one or more similar subjects that share similar characteristics to the target subject.

11. The computer-implemented method of claim 10, wherein the one or more characteristics of the target subject comprise one or more of: a level of engagement of the target subject, the needs of the target subject, an aimed value of care for the target subject, a susceptibility to conversion of the target subject, a cost of care available to the target subject, and/or informal caregiver availability and involvement.

12. The computer-implemented method (200) of any of claims 1 to 11, further comprising a step (240) of providing, at a user interface, a visual representation of the recommended journey.

13. A computer program product comprising computer program code means which, when executed on a computing device having a processing system, cause the processing system to perform all of the steps of the method according to any of claims 1 to 12.

14. A processing system (110, 60, 700) for generating information for guiding a target subject (190) through a care or treatment selection process, the processing system being configured to:
identify (210) one or more similar subjects to the target subject;
obtain (220) historic journey information comprising, for each similar subject, information about a journey taken by the subject during a care or treatment selection process, wherein a journey is a sequence of steps performed by the subject during a care or treatment selection process; and
process (230, 300, 400) the historic journey information to generate a recommended journey for the target subject.
